# EUROPÄISCHE PATENTSCHRIFT

(11) **EP 2 957 275 B1**
(45) Veröffentlichungstag und Bekanntmachung des Hinweises auf die Patenterteilung: **15.05.2019**
(21) Anmeldenummer: 15170682.7
(22) Anmeldetag: 04.06.2015
(51) Int. Cl.: A61K 8/9789, A61K 8/34, A61Q 19/02

(54) **KOSMETISCHE ODER DERMATOLOGISCHE ZUBEREITUNGEN MIT KOMBINATIONEN AUS 4-N-BUTYLRESORCIN UND MAGNOLIENRINDENEXTRAKT SOWIE EINEM ODER MEHREREN DIOLEN**
COSMETIC OR DERMATOLOGICAL PREPARATIONS WITH COMBINATIONS OF 4-N-BUTYLRESORCIN AND MAGNOLIA BARK EXTRACT AND ONE OR MORE DIOLS
PRÉPARATIONS COSMÉTIQUES OU DERMATOLOGIQUES AYANT DES COMBINAISONS D'EXTRAITS D'ÉCORCE DE MAGNOLIA ET DE 4-N-BUTYLRESORCINE ET D'UN OU PLUSIEURS DIOLES

(30) Priorität: 20.06.2014 DE 102014211825
(43) Veröffentlichungstag der Anmeldung: 23.12.2015
(73) Patentinhaber: Beiersdorf AG, 20253 Hamburg (DE)
(72) Erfinder: Breitkreutz, Sabrina, 22303 Hamburg (DE); Bölke, Diana, 22297 Hamburg (DE); Kionka, Julia, 15566 Schöneiche (DE); Bluck, Manuela, 21529 Kröppelshagen-Fahrendorf (DE); Möbius, Janne, 20257 Hamburg (DE)

(56) Entgegenhaltungen:
- EP-A1- 2 786 741
- EP-A2- 2 033 622
- WO-A1-2011/131445
- WO-A1-2013/081147
- WO-A2-2014/086544
- DE-A1-102012 211 797
- JP-A- 2008 156 260

## Beschreibung

Die vorliegende Erfindung betrifft kosmetische oder dermatologische Zubereitungen mit Kombinationen aus 4-n-Butylresorcin und/oder Mangnolienrindenextrakt sowie einem oder mehreren Diolen.

Für die Pigmentierung der Haut verantwortlich sind die Melanozyten, welche in der untersten Schicht der Epidermis, dem Stratum basale, neben den Basalzellen als - je nach Hauttyp entweder vereinzelt oder aber mehr oder weniger gehäuft auftretende - pigmentbildende Zellen vorzufinden sind.

Melanozyten enthalten als charakteristische Zellorganellen Melanosomen, in denen das Melanin gebildet wird. Unter anderem bei Anregung durch UV-Strahlung wird verstärkt Melanin gebildet. Dieses wird über die lebenden Schichten der Epidermis (Keratinozyten) letztlich in die Hornschicht (Corneozyten) transportiert und ruft eine mehr oder weniger ausgeprägte bräunliche bis braun-schwarze Hautfarbe hervor.

Melanin wird als Endstufe eines oxidativen Prozesses gebildet, in welchem Tyrosin unter Mitwirkung der Enzyms Tyrosinase über mehrere Zwischenstufen zu den braun bis braunschwarzen Eumelaninen (DHICA- und DHI-Melanin) bzw. unter Beteiligung von schwefelhaltigen Verbindungen zum rötlichen Phäomelanin umgewandelt wird. DHICA- und DHI-Melanin entstehen über die gemeinsamen Zwischenstufen Dopachinon und Dopachrom. Letzteres wird, teilweise unter Beteiligung weiterer Enzyme, entweder in Indol-5,6-Chinon-Carbonsäure oder in Indol-5,6-Chinon umgesetzt, woraus die beiden genannten Eumelanine entstehen.

Die Entstehung von Phäomelanin läuft unter anderem über die Zwischenprodukte Dopachinon und Cysteinyldopa. Gesteuert wird die Expression der Melanin-synthetisierenden Enzyme durch einen spezifischen Transkriptionsfaktor (microphthalmia- associated transcription factor, MITF). Neben den beschriebenen enzymatischen Prozessen der Melanin-Synthese sind in den Melanosomen noch weitere Proteine für die Melanogenese von Bedeutung. Eine wichtige Rolle scheint hier dem sogenannten p-Protein zuzukommen, wobei die exakte Funktion noch unklar ist.

Neben dem zuvor beschriebenen Prozess der Melanin-Synthese in den Melanozyten, ist bei der Pigmentierung der Haut auch der Transfer der Melanosomen, deren Verbleib in der Epidermis sowie deren Abbau und der Abbau des Melanins von entscheidender Bedeutung. Es konnte gezeigt werden, dass für den Transport der Melanosomen aus den Melanozyten in die Keratinozyten der PAR-2-Rezeptor bedeutsam ist (M. Seiberg et al., 2000, J. Cell. Sci., 113:3093-101).

Ferner haben Größe und Form der Melanosomen Einfluss auf ihre lichtstreuenden Eigenschaften und somit das farbliche Erscheinungsbild der Haut. So findet man bei Schwarzafrikanern verstärkt große spheroidale, einzeln vorliegende Melanosomen, während man bei Kaukasiern eher kleinere, in Gruppen vorkommende, Melanosomen vorfindet.

Probleme mit Hyperpigmentierung der Haut haben vielfältige Ursachen bzw. sind Begleiterscheinungen vieler biologischer Vorgänge, z. B. UV-Strahlung (z.B. Sommersprossen, *Ephelides*), genetische Disposition, Fehlpigmentierung der Haut bei der Wundheilung bzw. -vernarbung (postinflammatorische Hyperpigmentierung) oder der Hautalterung (z.B. *Lentigines seniles*).

Nach entzündlichen Reaktionen reagiert das Pigmentierungssystem der Haut mit teilweise entgegengesetzten Reaktionen. Es kann sowohl zu postinflammatorischen Hyper- wie auch Hypopigmentierungen kommen. Postinflammatorische Hypomelanosen treten u. a. häufig in Verbindung mit Atopie, Lupus erythematosus und Psoriasis auf. Die unterschiedlichen Reaktionsformen des Pigmentierungssystems der menschlichen Haut in Folge entzündlicher Erscheinungen sind nur sehr unvollständig verstanden.

Probleme mit postinflammatorischer Hyperpigmentierung treten häufig bei dunkleren Hauttypen auf. Insbesondere bei männlichen Farbigen ist das Problem der *Pseudofollikulitis barbae* bekannt, das mit kosmetisch unerwünschten Fehlpigmentierung einhergeht bzw. diese nach sich zieht. Auch Formen von Melasma, welche insbesondere bei Frauen asiatischer Zugehörigkeit im Gesicht und im Dekolleté-Bereich auftreten, sowie verschiedene Formen der unregelmäßigen Pigmentierung der Haut werden zu den postinflammatorischen Hyperpigmentierungen gezählt. Ferner werden auch dunkle Augenringe als eine Form postinflammatorischen Hyperpigmentierungen angesehen, wobei die zugrunde liegende Entzündung meist subklinisch abläuft.

In vielen Fällen werden derartige postinflammatorische Fehlpigmentierung durch Einwirkung von Sonnenlicht (UV-Licht) noch verstärkt, ohne dass es zu einer UV-induzierten Entzündung (Sonnenbrand) kommt.

Es sind Wirkstoffe und Zubereitungen bekannt, welche der Hautpigmentierung entgegenwirken. Im praktischen Gebrauch sind im Wesentlichen Präparate auf der Grundlage von Hydrochinon, welche aber einesteils erst nach mehrwöchiger Anwendung ihre Wirkung zeigen, deren übertrieben lange Anwendung andererseits aus toxikologischen Gründen bedenklich ist. Von Albert Kligman et al. wurde eine sogenannte Triformula entwickelt, die eine Kombination aus 0.1% Tretinoin, 5.0% Hydroquinone, 0.1% Dexamethasone darstellt (A. Kligman, 1975, Arch. Dermatol., 111:40-48). Allerdings ist auch diese Formulierung wegen möglicher irreversibler Veränderungen im Pigmentierungssystem der Haut sehr umstritten.

Ferner finden hautschälende Methoden (chemische und mechanische "Peelings") Anwendung, die jedoch häufig entzündliche Reaktionen nach sich ziehen und aufgrund danach eintretender postinflammatorischer Hyperpigmentierungen sogar zu stärkerer statt verminderter Pigmentierung führen können. All diese gängigen Verfahren, die auch zur Behandlung von postinflammatorischen Hypergigmentierungen angewendet werden, zeichnen sich durch entscheidende Nebenwirkungen aus.

Ziel der nachfolgenden Erfindung war es also, dem nachteiligen Stand der Technik Abhilfe zu verschaffen.

Magnolienrindenextrakt ist bekanntermaßen ein antimikrobiell wirksames Agens und wurde seit Langem in der indianischen Volksmedizin verwendet. Nachteilig ist, dass solche Extrakte leichtverderblich sind und sich bereits in kurzer Frist durch Lichteinfall und Luftzutritt dunkel zu verfärben beginnen, und ihre Wirksamkeit nimmt messbar ab.

Kosmetische Zusammensetzung mit Magnolienrindenextrakt, die durch eine oder mehrere ober- oder grenzflächenaktive polyethoxylierte Verbindungen stabilisiert sind, sind aus WO 2011/131445 bekannt. Eine weitere Aufgabe der vorliegenden Erfindung bestand darin, Wirkstoffe bzw. Zubereitungen, solche Wirkstoffe enthaltend, zur Verfügung zu stellen, die sich durch eine erhöhte Beständigkeit gegenüber Licht- und/oder Lufteinwirkung auszeichnen würden.

Übliche kosmetische Darreichungsformen sind Emulsionen. Darunter versteht man im allgemeinen ein heterogenes System aus zwei miteinander nicht oder nur begrenzt mischbaren Flüssigkeiten, die üblicherweise als Phasen bezeichnet werden. Die eine liegt dabei in Form von Tröpfchen vor (dispere oder innere Phase), während die andere Flüssigkeit eine kontinuierliche (kohärente oder innere Phase) bildet. Seltenere Darreichungsformen sind multiple Emulsionen, also solche, welche in den Tröpfchen der dispergierten (oder diskontinuierlichen) Phase ihrerseits Tröpfchen einer weiteren dispergierten Phase enthalten, z.B. W/O/W-Emulsionen und O/W/O-Emulsionen.

Neuere Erkenntnisse führten in letzter Zeit zu einem besseren Verständnis praxisrelevanter kosmetischer Emulsionen. Dabei geht man davon aus, dass die im Überschuss eingesetzten Emulgatorgemische lamellare flüssigkristalline Phasen bzw. kristalline Gelphasen ausbilden. In der Gelnetzwerktheorie werden Stabilität und physikochemische Eigenschaften solcher Emulsionen auf die Ausbildung von viskoelastischen Gelnetzwerden zurückgeführt. Kosmetische Zubereitungen mit 4-n-Butylresorcin sind bekannt, beispielsweise aus der EP 1 490 017.

4-n-Butylresorcin wird auch Rucinol oder Lucinol genannt. Es hemmt die Melaninproduktion, indem es das für die Melaninsynthese (Melanogenese) erforderliche Enzym, die Tyrosinase, hemmt. Es wird zunächst die Melaninproduktion gehemmt, dann die Produktion des schwarzen Melanins, das für die intensive Färbung der Pigmentflecken verantwortlich ist, blockiert. Obwohl die Verwendung von 4-n-Butylresorcin kosmetisch zweifellos vorteilhaft wäre, sind kosmetische Zubereitungen mit einem Gehalt an 4-n-Butylresorcin schwierig zu stabilisieren, da dieses empfindlich gegen Oxidation ist. Dies macht sich durch verschiedene Erscheinungen bemerkbar, unter anderem die, dass sich Zubereitungen mit 4-n-Butylresorcin schnell bräunlich verfärben.

Es war also eine Aufgabe der vorliegenden Erfindung, diesem Übelstand abzuhelfen.

Es war überraschend und für den Fachmann nicht vorhersehbar, dass Übelstände des Standes der Technik beseitigt werden durch kosmetische oder dermatologische Zubereitungen mit Kombinationen aus
i) 4-n-Butylresorcin
   und
ii) einem mit Hilfe von superkritischem CO₂ oder Ethanol oder einer Ethanol / Wasser Mischung gewonnenen Magnolienrindenextrakt aus Magnolia grandiflora mit einem Gehalt an Magnolol und/oder Honokiol, sowie
iii) einem oder mehreren Diolen aus der Gruppe 1,2-Octandiol, und Octoxyglycerin (2-Ethylhexylglycerinether). 4-n-Butylresorcin, CAS [18979-61-8], ist durch die chemische Struktur 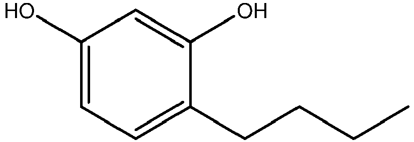 gekennzeichnet.

Bevorzugt enthalten kosmetische oder dermatologische Zubereitungen gemäß der Erfindung 0,001 - 10 Gew.-%, besonders bevorzugt 0,01 - 1 Gew.-%, an 4-n-Butylresorcin, bezogen auf die Gesamtzusammensetzung der Zubereitungen.

Magnolien (Magnolia) sind eine Pflanzengattung in der Familie der Magnoliengewächse (Magnoliaceae). Diese enthält etwa 230 Arten, die aus Ostasien und Amerika stammen. Erfindungsgemäß kann der Magnolienrindenextrakt grundsätzlich allen Arten der Gattung Magnolia entnommen werden, wobei allerdings Magnolia grandiflora, und insbesondere Magnolia officinalis bevorzugt werden.

Magnolien-Rinde wird in der Traditionellen Chinesischen Medizin (TCM) vor allem bei "Stagnation of qi" (low energy), emotionalem Stress, Entzündungen, innerer Unruhe und Angstzuständen eingesetzt.

Die Herstellung des verwendeten Extraktes erfolgt mit Hilfe von superkritischem CO₂ oder Ethanol oder einer Ethanol / Wasser Mischung.

Als typische Inhaltstoffe und Leitsubstanzen Leitsubstanzen des Extraktes sind Magnolol und Honokiol definiert.

Magnolol ist durch folgende Struktur gekennzeichnet:

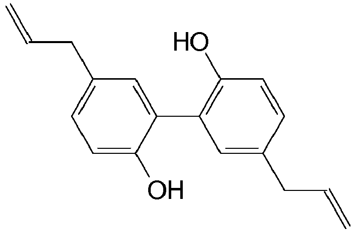

Honokiol ist durch folgende Struktur gekennzeichnet:

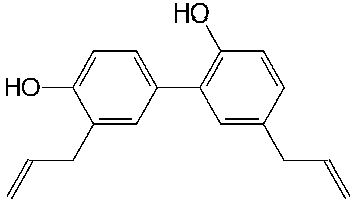

Die erfindungsgemäßen kosmetischen oder dermatologischen Zubereitungen enthalten bevorzugt 0,001 Gew.-% bis 10 Gew.-%, bevorzugt 0,005 Gew.-% bis 5 Gew.-%, insbesondere 0,01 - 2,0 Gew.-%, bezogen auf das Gesamtgewicht der Zubereitungen, an Mangnolienrindenextrakt.

Die erfindungsgemäßen Zubereitungen zeichnen sich durch eine hohe Wirksamkeit bei gleichzeiter sehr guter Hautverträglichkeit aus. Außerdem haben sie ausgezeichnete sensorische Eigenschaften wie ein geringes Klebrigkeitsgefühl auf der Haut.

Die erfindungsgemäß besonders bevorzugten Diole sind 1,2-Octandiol, und Octoxyglycerin (2-Ethylhexylglycerinether).

Die erfindungsgemäßen Zubereitungen sind besonders vorteilhaft dadurch gekennzeichnet, dass das oder die Diole in Konzentrationen von 0,01 - 20,00 Gew.-%, bevorzugt 0,1 - 10,00 Gew.-% jeweils bezogen auf das Gesamtgewicht der Zusammensetzung, vorliegt oder vorliegen.

Erfindungsgemäß werden die Kombinationen aus den unter den Punkten i) bis iii) genannten Substanzen bevorzugt in kosmetischen oder dermatologischen Zusammensetzungen eingesetzt einem Gehalt von 0,012 - 40,0 Gew.-%, insbesondere 0,012 - 20,0 Gew.-%, bezogen auf das Gesamtgewicht der Zusammensetzung bevorzugt sind. Besonders vorteilhaft enthalten die Zusammensetzungen 0,02 - 10,0 Gew.-%, ganz besonders bevorzugt sind 0,05 - 5,0 Gew.-% jeweils bezogen auf das Gesamtgewicht der Zusammensetzung.

Zur Anwendung werden die kosmetischen und dermatologischen Zubereitungen erfindungsgemäß in der für Kosmetika üblichen Weise auf die Haut und/oder die Haare in ausreichender Menge aufgebracht.

Besonders bevorzugt sind solche kosmetischen und dermatologischen Zubereitungen, die in der Form eines Sonnenschutzmittels vorliegen. Vorteilhaft können diese zusätzlich mindestens einen weiteren UVA-Filter und/oder mindestens einen weiteren UVB-Filter und/oder mindestens ein anorganisches Pigment, bevorzugt ein anorganisches Mikropigment, enthalten.

Die kosmetischen und dermatologischen Zubereitungen können erfindungsgemäß kosmetische Hilfsstoffe enthalten, wie sie üblicherweise in solchen Zubereitungen verwendet werden, z. B. Konservierungsmittel, Bakterizide, Parfüme, Substanzen zum Verhindern des Schäumens, Farbstoffe, Pigmente, die eine färbende Wirkung haben, Verdickungsmittel, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Ein zusätzlicher Gehalt an üblichen Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Die Menge der vorgenannten Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern die kosmetische oder dermatologische Zubereitung im Sinne der vorliegenden Erfindung eine Lösung oder Emulsion oder Dispersion darstellt, können als Lösungsmittel bzw als Ölkörper verwendet werden:
- Wasser oder wässrige Lösungen
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Sofern die kosmetische oder dermatologische Zubereitung im Sinne der vorliegenden Erfindung eine Lösung oder Emulsion oder Dispersion darstellt, können als Lösungsmittel verwendet werden:
- Wasser oder wässrige Lösungen
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Als Treibmittel für aus Aerosolbehältern versprühbare kosmetische und/oder dermatologische Zubereitungen im Sinne der vorliegenden Erfindung sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, dass es an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Kosmetische Zubereitungen im Sinne der vorliegenden Erfindung können auch als Gele vorliegen, die neben einem wirksamen Gehalt am erfindungsgemäßen Wirkstoff und dafür üblicherweise verwendeten Lösungsmitteln, bevorzugt Wasser, noch organische Verdickungsmittel, z. B. Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z. B. Aluminiumsilikate wie beispielsweise Bentonite, oder ein Gemisch aus Polyethylenglykol und Polyethylenglykolstearat oder -distearat, enthalten. Das Verdickungsmittel ist in dem Gel z.B. in einer Menge zwischen 0,1 und 30 Gew.-%, bevorzugt zwischen 0,5 und 15 Gew.-%, enthalten.

Es ist erfindungsgemäß vorteilhaft, außer den erfindungsgemäßen Kombinationen weitere öllösliche UVA-Filter und/oder UVB-Filter in der Lipidphase und/oder weitere wasserlösliche UVA-Filter und/oder UVB-Filter in der wässrigen Phase einzusetzen.

Vorteilhaft können die Lichtschutzformulierungen erfindungsgemäß weitere Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen, um kosmetische Zubereitungen zur Verfügung zu stellen, die die Haut vor dem gesamten Bereich der ultravioletten Strahlung schützen. Sie können auch als Sonnenschutzmittel dienen.

Der Parameter "Verfärbung" soll mit einer Messmethode definiert werden. Ein handelsüblicher "spectro-pen" misst drei Werte, die den sogenannten L*a*b-Farbraum aufspannen und Auskunft über Helligkeit und den Farbraum geben, indem die ermittelten Werte miteinander verglichen werden.

Jede wahrnehmbare Farbe im Farbraum ist durch den Farbort mit den Koordinaten {L*, a*, b*} auf 3 Achsen definiert. Die Werte der Helligkeit beschreibt der ΔL-Wert (Endpunkte: schwarz und weiß, beschreibt die Grautöne). Die Farben Grün und Rot werden durch den a*-Wert, die Farben Blau und Gelb durch den b*-Wert beschrieben.

### Der L*a*b*-Farbraum

Der L*a*b*-Farbraum ist ein Messraum, in dem alle wahrnehmbaren Farben enthalten sind. Eine der wichtigsten Eigenschaften des L*a*b*-Farbmodells ist seine Geräteunabhängigkeit, das heißt, die Farben werden unabhängig von der Art ihrer Erzeugung und Wiedergabetechnik definiert.

Die entsprechende deutsche Norm ist *DIN 6174*: "Farbmetrische Bestimmung von Farbmaßzahlen und Farbabständen im angenähert gleichförmigen CIELAB-Farbenraum".[1]

Der so standardisierte Farbraum ist wie beschrieben gleichabständig und geräteunabhängig. Jede wahrnehmbare Farbe im Farbraum ist durch den Farbort mit den Koordinaten {L*, a*, b*} definiert.

Der L*a*b*-Farbraum wird durch ein dreidimensionales Koordinatensystem beschrieben. Die a*-Achse beschreibt den Grün- oder Rotanteil einer Farbe, wobei negative Werte für Grün und positive Werte für Rot stehen. Die b*-Achse beschreibt den Blau- oder Gelbanteil einer Farbe, wobei negative Werte für Blau und positive Werte für Gelb stehen. Die Skalen der a*-Achse und der b*-Achse umfassen einen Zahlenbereich von -150 bis +100 und -100 bis +150, ungeachtet dessen, dass es für einige Werte keine wahrnehmbare Entsprechung gibt.

Die L*-Achse steht auf diesen Ebenen senkrecht und gibt die Helligkeit (Luminanz) der Farben mit Werten von 0 bis 100 wieder. Die L*-Achse kann auch als Neutralgrauachse bezeichnet werden, da sie die Endpunkte Schwarz (L=0) und Weiß (L=100) besitzt und die Zwischenwerte auf dieser Achse die unbunten Grautöne sind.

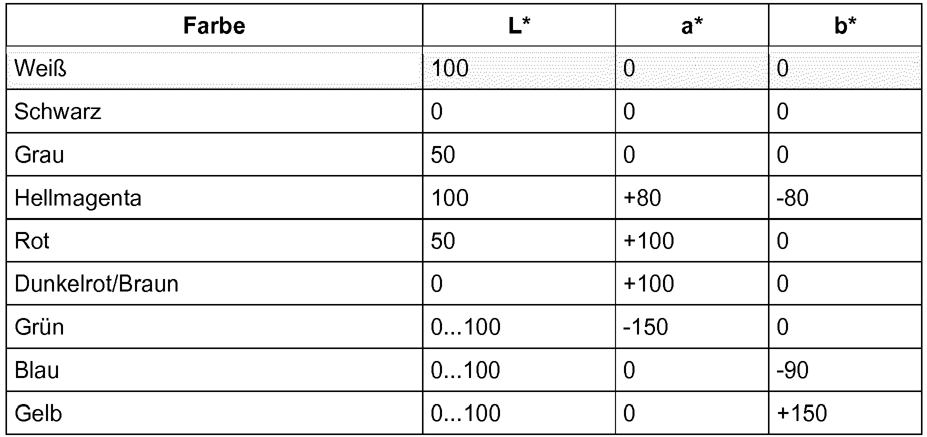

Quelle: http://de.wikipedia.org/wiki/Lab-Farbraum
[1] Beuth-Verlag: DIN 6174: Farbmetrische Bestimmung von Farbmaßzahlen und Farbabständen im angenähert gleichförmigen CIELAB-Farbenraum (Ausgabe *DIN 6174:2007-10)*

### Messgerät und Durchführung:

spectro-pen® , Firma: Dr. Bruno Lange GmbH und Co. KG
Typ Nr. LMG161

### Versuchsdurchführung:

Die zu messenden Probe wird in ein Vial mit einem glatten Boden ("Wheaton liquid scintillation vials", Glas, 20ml) mit definierter Füllhöhe (die zu vergleichenden Proben sollten dieselbe Füllhöhe besitzen) vermessen. Hierzu wird der spectro-pen® am Glasboden das Glases luftdicht an das Glas gehalten und es wird vom Gerät der Mittelwert aus den gemessenen 3 Einzelmessungen der L*a* b*-Werte berechnet.

Die für die Erfindng relevanten a- und b-Werte sind in den folgenden Tabellen aufgezeigt. Beispiele, die nicht in den Bereich der Ansprüche fallen, sind als Referenzbeispiele zu betrachten. :

**Messwerte für Ansätze mit Wirkstofflösung 1**

| | **Lagerort** | **a** | **\|Δa\|** | **b** | **\|Δb\|** |
|---|---|---|---|---|---|
| A1 | 40°C | 3,5 | | 9,6 | |
| Referenz ohne Diole | Licht | -3,5 | | 9 | |
| B1 | 40°C | 3,4 | 0,1 | 9,2 | 0,4 |
| 1% 2-Methylpropandiol | Licht | -2 | 1,5 | 7,5 | 1,5 |
| C1 | 40°C | 2,4 | 1,1 | 8,4 | 1,2 |
| 2% 2-Methylpropandiol | Licht | -1,9 | 5,4 | 6,3 | 2,7 |
| D1 | 40°C | 0,4 | 2,1 | 6,7 | 2,9 |
| 0.25% Octoxyglycerin | Licht | -4,1 | 0,6 | 5,1 | 4,5 |
| E1 | 40°C | 4,9 | 1,4 | 8,4 | 1,2 |
| 0.25% 1,2-Hexandiol | Licht | -1,4 | 2,1 | 7,6 | 1,4 |
| F1 | 40°C | 4,8 | 1,3 | 6 | 3,6 |
| 0,25% 1,2-Octandiol | Licht | -2,4 | 1,1 | 6,5 | 2,5 |
| D3 | 40°C | -0,9 | 2,6 | 6,5 | 3,1 |
| 0.5% Octoxyglycerin | Licht | -3,9 | 0,4 | 5,2 | 3,8 |
| E3 | 40°C | 5,2 | 1,7 | 7,1 | 2,5 |
| 0.5% 1,2-Hexandiol | Licht | -1,2 | 2,3 | 7,7 | 1,3 |
| F3 | 40°C | 3 | 0,5 | 7,2 | 2,4 |
| 0,5% 1,2-Octandiol | Licht | -3,3 | 0,2 | 4,8 | 4,2 |

**Messwerte für Ansätze mit Wirkstofflösung 2:**

| | **Lagerort** | **a** | **\|Δa\|** | **b** | **\|Δb\|** |
|---|---|---|---|---|---|
| A2 | 40°C | 18 | | 10,6 | |
| Referenz ohne Diole | Licht | 2,2 | | 3,7 | |
| B2 | 40°C | 22 | 4 | 13,6 | 4 |
| 1% 2-Methylpropandiol | Licht | 2,3 | 0,1 | -1,3 | 5 |
| C2 | 40°C | 14 | 4 | 6,9 | 3,7 |
| 2% 2-Methylpropandiol | Licht | 2,7 | 0,5 | -0,5 | -4,2 |
| D2 | 40°C | 13 | 5 | 10,1 | 0,5 |
| 0.25% Octoxyglycerin | Licht | -0,1 | 2,3 | 0,5 | 3,2 |
| E2 | 40°C | 16 | 2 | 8,4 | 2,2 |
| 0.25% 1,2-Hexandiol | Licht | 2,2 | 0 | -1,1 | 4,8 |
| F2 | 40°C | 10 | 8 | 4,4 | 6,2 |
| 0,25% 1,2-Octandiol | Licht | 0,6 | 1,6 | -1,5 | 5,2 |
| G2 | 40°C | 7,2 | 11 | 8,1 | 2,5 |
| 2% 2-Methylpropandiol 0.25% Octoxyglycerin | Licht | -0,5 | -2,7 | -0,8 | 4,5 |
| H2 | 40°C | 12 | 5,8 | 5,5 | 5,1 |
| 2% 2-Methylpropandiol 0.25% 1,2-Hexandiol | Licht | 3 | 0,8 | 0,6 | 3,1 |
| I2 | 40°C | 17 | 1,3 | 9,9 | 0,7 |
| 2% 2-Methylpropanediol 0,25% 1,2-Octandiol | Licht | 1,5 | 0,7 | -1,8 | 5,5 |
| D4 | 40°C | 4,7 | 13 | 7,2 | 3,4 |
| 0.5% Octoxyglycerin | Licht | -0,4 | 2,6 | 0,2 | 3,5 |
| E4 | 40°C | 8,8 | 9 | 2,8 | 7,8 |
| 0.5% 1,2-Hexandiol | Licht | 2,3 | 0,1 | 1 | 2,7 |
| F4 | 40°C | 5,9 | 12 | 1,9 | 8,7 |
| 0,5% 1,2-Octandiol | Licht | -0,2 | 2,4 | -1,7 | 5,4 |

Wie aus der Tabelle ersichtlich ist, kann die Verfärbung der Wirkstofflösungen der jeweiligen Referenz ohne Diole Ansatz A1 bzw. A2 durch die jeweiligen Diole bei 40°C- und/ oder Licht-Lagerung deutlich minimiert werden.

Als Verbesserung im Sinne der vorliegenden Verfärbung kann der absolute Unterschied |Δa|≥2 und/ oder |Δb|≥2 - die entsprechenden Rot-Grün- bzw. Gelb-Blauanteile einer Farbeangesehen werden. Je größer der ermittelte absolute Unterschied, desto stärker der Farbunterschied.

### Versuchsbeschreibung

Es wurden Wirkstofflösungen mit unterschiedlichen Konzentrationen an Magnolienrindenextrakt und 4-n-Butylresorcin und Ethanol hergestellt.

| Wirkstofflösung | **1** | **2** |
|---|---|---|
| Einwaage | g | g |
| Ethanol | 100 | 100 |
| Magnolienrindenextrakt | 0,5 | 2,5 |
| 4-n-Butylresorcin | 0,1 | 0,5 |

Für die Wirkstoff- und Diol-haltigen Lösungen wurde die enstprechende Wirkstofflösung vorgelegt, das/ die Diol/e hinzugewogen und mit Wasser auf 100% ergänzt. Die folgende Tabelle stellt die hergestellten Rezepturen dar:

| Rezepturbestandteile: | | Ansatz | | | | | |
|---|---|---|---|---|---|---|---|
| | Gew.-% | A1 | B1 | C1 | D1 | E1 | F1 |
| Wirkstofflösung **1** | 10,06 | x | x | x | x | x | x |
| 2-Methylpropandiol | 1 | | x | | | | |
| 2-Methylpropandiol | 2 | | | x | | | |
| Octoxyglycerin | 0,25 | | | | x | | |
| 1,2-Hexandiol | 0,25 | | | | | x | |
| 1,2-Octandiol | 0,25 | | | | | | x |
| Wasser | ad 100 | x | x | x | x | x | x |

| Rezepturbestandteile: | | Ansatz | | | | | | | | |
|---|---|---|---|---|---|---|---|---|---|---|
| | Gew.-% | A2 | B2 | C2 | D2 | E2 | F2 | G2 | H2 | I2 |
| Wirkstofflösung **2** | 10,3 | x | x | x | x | x | x | x | x | x |
| 2-Methylpropandiol | 1 | | x | | | | | | | |
| 2-Methylpropandiol | 2 | | | x | | | | x | x | x |
| Octoxyglycerin | 0,25 | | | | x | | | x | | |
| 1,2-Hexandiol | 0,25 | | | | | x | | | x | |
| 1,2-Octandiol | 0,25 | | | | | | x | | | x |
| Wasser | ad 100 | x | x | x | x | x | x | x | x | x |

| Rezepturbestandteile: | | Ansatz | | |
|---|---|---|---|---|
| | Gew.-% | D3 | E3 | F3 |
| Wirkstofflösung **1** | 10,06 | x | x | x |
| 2-Methylpropandiol | 1 | | | |
| 2-Methylpropandiol | 2 | | | |
| Octoxyglycerin | 0,5 | x | | |
| 1,2-Hexandiol | 0,5 | | x | |
| 1,2-Octandiol | 0,5 | | | x |
| Wasser | ad 100 | x | x | x |

| Rezepturbestandteile: | | Ansatz | | |
|---|---|---|---|---|
| | Gew.-% | D4 | E4 | F4 |
| Wirkstofflösung **2** | 10,3 | x | x | x |
| 2-Methylpropandiol | 1 | | | |
| 2-Methylpropandiol | 2 | | | |
| Octoxyglycerin | 0,5 | x | | |
| 1,2-Hexandiol | 0,5 | | x | |
| 1,2-Octandiol | 0,5 | | | x |
| Wasser | ad 100 | x | x | x |

Die hergestellten Lösungen wurden in "Wheaton liquid scintillation vials", Glas, 20ml) mit definierter Füllhöhe (die zu vergleichenden Proben sollten dieselbe Füllhöhe besitzen) gefüllt und bei 40°C und am Licht 7d gelagert und anschließend vermessen.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen. Beispiele, die nicht in den Bereich der Ansprüche fallen, sind als Referenzbeispiele zu betrachten.

| **Beispiele** | **1** | **2** | **3** | **4** | **5** | **6** |
|---|---|---|---|---|---|---|
| INCI-Bezeichnung | Gew.-% | Gew.-% | Gew.-% | Gew.-% | Gew.-% | Gew.-% |
| | | | | | | |
| 4-n-Butyl-resorcinol | 0,2 | 0,3 | 0,25 | 0,3 | 0,3 | 0,5 |
| Magnolienrindenextrakt | 0,25 | 0,1 | 0,05 | 0,1 | 0,1 | 0,25 |
| Mica | 0,5 | 0 | 0 | 0,5 | 0,5 | 0 |
| Cetylstearyl Alkohol | 0,75 | 1,5 | 0,75 | 1,5 | 2 | 1,5 |
| Dimethicon | 5 | 4 | 4 | 4 | 5 | 5 |
| Cyclomethicon | 10 | 10 | 10 | 10 | 10 | 10 |
| Natrium Stearoyl Glutamat | 0,2 | 0,2 | 0,2 | 0,2 | 0 | 0,2 |
| Natrium Cetystearyl Sulfat | 0 | 0 | 0 | 0 | 0,4 | 0 |
| Glyceryl Stearat | 0 | 0 | 0 | 0 | 3 | 0 |
| Tapioca Stärke | 0 | 2 | 0 | 2 | 2 | 0 |
| Parfum | 0,4 | 0,4 | 0,4 | 0,3 | 0,5 | 0,5 |
| Glycerin | 7 | 9 | 4 | 9 | 9 | 12 |
| Phenoxyethanol | 0,8 | 0,8 | 0,8 | 0,6 | 0,6 | 0,8 |
| 1,2-Octandiol | 0 | 0 | 0 | 0 | 0 | 0,3 |
| 1,2-Hexandiol | 0,5 | 0 | 0,7 | 0 | 0 | 0 |
| Octoxyglycerin | 0 | 0,25 | 0 | 0,25 | 0,25 | 0,25 |
| 2-Methylpropandiol | 2 | 3 | 0 | 2 | 2 | 3 |
| Carbomer | 0,2 | 0,3 | 0,2 | 0,4 | 0,3 | 0,2 |
| Acrylates/C10-30 Alkyl Acrylat Cross-polymer | 0,1 | 0,1 | 0,1 | 0,1 | 0,1 | 3 |
| Caprylic/Capric Triglycerid | 1 | 0,5 | 0,1 | 1 | 0,5 | 1 |
| Natriumhydroxid | q.s | q.s | q.s | q.s | q.s | q.s |
| Wasser | ad 100 | ad 100 | ad 100 | ad 100 | ad 100 | ad 100 |

| **Beispiele** | **7** | **8** | **9** | **10** | **11** | **12** |
|---|---|---|---|---|---|---|
| INCI-Bezeichnung | Gew.-% | Gew.-% | Gew.-% | Gew.-% | Gew.-% | Gew.-% |
| | | | | | | |
| 4-n-Butylresorcin | 0,25 | 0,25 | 0,1 | 0,1 | 0,1 | 0,25 |
| Magnolienrindenextrakt | 0,25 | 0,25 | 0,3 | 0,3 | 0,3 | 0,25 |
| Cetylstearyl Alkohol | 1 | 1 | 2,5 | 2,5 | 2,5 | 2,5 |
| C12-15 Alkyl Benzoate | 2 | 3 | 2 | 1 | 2 | 1 |
| Glyceryl Stearat | 0 | 0 | 2 | 1,5 | 2 | 1,5 |
| Glyceryl Stearate Citrat | 2,5 | 2,25 | 0 | 0 | 0 | 0 |
| Glyceryl Stearat SE | 0 | 0 | 0 | 0 | 3 | 0 |
| Stearinsäure | 0 | 0 | 3 | 2,75 | 0 | 2,75 |
| Polymethylsilsesquioxan | 0 | 1 | 0 | 1 | 0,5 | 1 |
| Dimethicon | 3 | 2,5 | 0 | 1 | 1 | 1 |
| Parfum | 0,3 | 0,3 | 0,5 | 0,5 | 0,5 | 0,5 |
| Glycerin | 10 | 9 | 10 | 9 | 9 | 9 |
| Phenoxyethanol | 0,8 | 0,8 | 0,8 | 0,8 | 0,8 | 0,8 |
| 1,2-Hexandiol | 0 | 0,5 | 0,3 | 0 | 0 | 0 |
| 1,2-Octandiol | 0 | 0 | 0 | 0 | 0 | 0,15 |
| Octoxyglycerin | 0,5 | 0,2 | 0,15 | 0,5 | 0,5 | 0,5 |
| 2-Methylpropandiol | 2 | 3 | 1 | 1,5 | 1,5 | 1,5 |
| Carbomer | 0,25 | 0,3 | 0,1 | 0,2 | 0,2 | 0,2 |
| Xanthan Gummi | 0,15 | 0 | 0,1 | 0,4 | 0,4 | 0,4 |
| Alkohol | 0 | 2 | 0 | 0 | 2 | 3 |
| Synthetisches Fluorphlogopit | 1 | 0,5 | 0 | 0 | 0 | 0 |
| CI77891 | 0 | 1 | 0 | 1 | 0,5 | 1 |
| Trinatrium EDTA | 0,1 | 0,1 | 0,15 | 0,15 | 0,15 | 0,15 |
| Octocrylen | 1,75 | 2,25 | 2 | 7 | 3 | 3 |
| Ethylhexylsalicylat | 5 | 4 | 5 | 5 | 4 | 4,5 |
| Homosalat | 0 | 0 | 0 | 4 | 4 | 0 |
| Butylmethoxydibenzoylmethan | 2,5 | 3 | 2,25 | 3 | 3 | 2 |
| Phenylbenzimidazolsulfonsäure | 1,5 | 1,5 | 0,75 | 0 | 0,8 | 0,75 |
| Natriumhydroxid | q.s | q.s | q.s | q.s | q.s | q.s |
| Wasser | ad 100 | ad 100 | ad 100 | ad 100 | ad 100 | ad 100 |

## Patentansprüche

1. Kosmetische oder dermatologische Zubereitungen mit Kombinationen aus
i) 4-n-Butylresorcin
und
ii) einem mit Hilfe von superkritischem CO₂ oder Ethanol oder einer Ethanol / Wasser Mischung gewonnenen Magnolienrindenextrakt aus Magnolia grandiflora mit einem Gehalt an Magnolol und/oder Honokiol,
sowie
iii) einem oder mehreren Diolen aus der Gruppe 1,2-Octandiol, und Octoxyglycerin (2-Ethylhexylglycerinether).

2. Zubereitungen nach Anspruch 1, **dadurch gekennzeichnet, daß** sie in Form von Emulsionen vorliegen.

3. Zubereitung nach Anspruch 1 oder 2, **dadurch gekennzeichnet, daß** die Zubereitungen 0,001 - 10 Gew.-%, besonders bevorzugt 0,01 - 1 Gew.-%, an 4-n-Butylresorcin enthalten, bezogen auf die Gesamtzusammensetzung der Zubereitungen.

4. Zubereitungen nach einem der vorstehenden Ansprüche, **dadurch gekennzeichnet, daß** sie 0,001 Gew.-% bis 10 Gew.-%, bevorzugt 0,005 Gew.-% bis 5 Gew.-%, insbesondere 0,01 - 2,0 Gew.-%, enthalten, bezogen auf das Gesamtgewicht der Zubereitungen, an Mangnolienrindenextrakt.

5. Zubereitungen nach einem der vorstehenden Ansprüche, **dadurch gekennzeichnet, daß** das oder die Diole in Konzentrationen von 0,01 - 20,00 Gew.-%, bevorzugt 0,1 - 10,00 Gew.-% jeweils bezogen auf das Gesamtgewicht der Zusammensetzung, vorliegt oder vorliegen.

## Claims

1. Cosmetic or dermatological preparations with combinations of
(i) 4-n-butylresorcinol
and
(ii) a magnolia bark extract of Magnolia grandiflora, having a content of magnolol and/or honokiol, obtained with the aid of supercritical CO₂ or ethanol or an ethanol/water mixture,
and
iii) one or more diols from the group of 1,2-octanediol and octoxyglycerol (2-ethylhexylglycerol ether).

2. Preparations according to Claim 1, **characterized in that** said preparations are present in the form of emulsions.

3. Preparation according to Claim 1 or 2, **characterized in that** the preparations comprise 0.001 - 10% by weight, particularly preferably 0.01 - 1% by weight. of 4-n-butylresorcinol, based on the total composition of the preparations.

4. Preparations according to any of the preceding claims, **characterized in that** said preparations comprise 0.001% by weight to 10% by weight, preferably 0.005% by weight to 5% by weight, especially 0.01 - 2.0% by weight of magnolia bark extract, based on the total weight of the preparations.

5. Preparations according to any of the preceding claims, **characterized in that** the diol or the diols is or are present at concentrations of 0.01 - 20.00% by weight, preferably 0.1 - 10.00% by weight, based in each case on the total weight of the composition.

## Revendications

1. Préparations cosmétiques ou dermatologiques, présentant des combinaisons constituées par
i) du 4-n-butylrésorcinol et
ii) un extrait d'écorce de magnolia obtenu à l'aide de CO₂ supercritique ou d'éthanol ou d'un mélange éthanol/eau à partir de Magnolia grandiflora, présentant une teneur en magnolol et/ou en honokiol, ainsi que
iii) un ou plusieurs diols du groupe constitué par le 1,2-octanediol et l'octoxyglycérol (2-éthylhexylglycéroléther).

2. Préparations selon la revendication 1, **caractérisées en ce qu'**elles se trouvent sous forme d'émulsions.

3. Préparation selon la revendication 1 ou 2, **caractérisée en ce que** les préparations contiennent 0,001-10 % en poids, de manière particulièrement préférée 0,01 - 1 % en poids, de 4-n-butylrésorcinol, par rapport à la composition totale des préparations.

4. Préparations selon l'une quelconque des revendications précédentes, **caractérisées en ce qu'**elles contiennent 0,001 % en poids à 10 % en poids, de préférence 0,005 % en poids à 5 % en poids, en particulier 0,01 - 2,0 % en poids, par rapport au poids total des préparations, d'extrait d'écorce de magnolia.

5. Préparations selon l'une quelconque des revendications précédentes, **caractérisées en ce que** le ou les diols se trouve(nt) en des concentrations de 0,01 - 20,00 % en poids, de préférence de 0,1 - 10,00 % en poids, à chaque fois par rapport au poids total de la composition.
